(19) Europäisches Patentamt
European Patent Office
Office européen des brevets

(11) **EP 4 481 421 A1**

(12) **EUROPEAN PATENT APPLICATION**

(43) Date of publication:
**25.12.2024 Bulletin 2024/52**

(21) Application number: **23180827.0**

(22) Date of filing: **22.06.2023**

(51) International Patent Classification (IPC):
**G01R 33/54** (2006.01)  **G01R 33/28** (2006.01)

(52) Cooperative Patent Classification (CPC):
**G01R 33/543; G16H 40/63;** G01R 33/288

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Koninklijke Philips N.V.
5656 AG Eindhoven (NL)**

(72) Inventor: **HOCK, Andreas
Eindhoven (NL)**

(74) Representative: **Philips Intellectual Property &
Standards
High Tech Campus 52
5656 AG Eindhoven (NL)**

(54) **MONITORING A GROUP OF DIAGNOSTIC IMAGING SYSTEMS**

(57) An arrangement (1) for monitoring a group of diagnostic imaging systems (15), the arrangement comprises a sensor system (12) for measurement of data representative of a multi-dimensional operational state space vectors for the individual diagnostic imaging systems. A status evaluation system (13) evaluates from the measured state space vectors, the location of the actual state space status of the diagnostic imaging systems in the multi-dimensional state space representing the diagnostic imaging systems' operational status and from the location in the multi-dimensional state space recognise an associated acceptable level of autonomous operation of the individual diagnostic imaging systems. A controller (14) operates to activate an action to control the individual diagnostic imaging systems to operate at the acceptable level of automation.

FIG. 1

EP 4 481 421 A1

**Description**

FIELD OF THE INVENTION

The invention pertains to an arrangement for monitoring a diagnostic imaging system. Notably, the invention aims at monitoring progress of acquisition of imaging data by a tomographic diagnostic imaging system such a as a magnetic resonance examination system, a computer-tomography system or nuclear medicine imaging (PET or SPECT) system.

BACKGROUND OF THE INVENTION

Such an arrangement is known from the European patent application EP 3 564 962.
The European patent application EP 3 564 962 discloses a magnetic resonance imaging system that comprises a machine learning model to predict a motion artifact level of the acquired magnetic resonance imaging data. The known magnetic resonance imaging system is configured to perform a re-start of a time-consuming data acquisition process from a very early stage, if artifacts are detected during the data acquisition.

SUMMARY OF THE INVENTION

An object of the present invention is to monitor a group of diagnostic imaging systems, so as to a larger extent enable the diagnostic imaging systems to operate in a (semi) autonomous manner. This object is achieved by the arrangement for monitoring a group of diagnostic imaging systems, the arrangement comprising

a sensor system for measurement of data representative of a multi-dimensional operational state space vectors for the individual diagnostic imaging systems;
a status evaluation system for evaluation from the measured state space vectors, the location of the actual state space status of the diagnostic imaging systems in the multi-dimensional state space representing the diagnostic imaging systems' operational status and from the location in the multi-dimensional state space recognise an associated acceptable level of autonomous operation of the individual diagnostic imaging systems and
a controller to activate an action to control the individual diagnostic imaging systems to operate at the acceptable level of automation.

The sensor system is configured to sense the status of the diagnostic imaging system with the patient to be examined when in operation. The sensed status may concern aspects of the status of the patient to be examined, status aspects of the imaging process and operational aspects of the operation of the diagnostic imaging system. The sensor system is configured measure physical and technical data of these aspects. The measured physical and technical data represent a state space vector of the operational state of the diagnostic imaging system with the patient to be examined. This operational state space vector's dimensions represent various technical functions of the diagnostic imaging system as well as physiological data of a subject in the examination zone (e.g. a patient to be examined in the bore of the magnetic resonance examination system or in the gantry of the computer-tomography system). The state space vector ($SV$) is typically built-up from the various component quantities and has for example for a magnetic resonance examination system the form:

$$SV = \begin{pmatrix} Im \\ Sys \\ Phys \\ ... \end{pmatrix}.$$

Here *Im* represents a sub-space vector relating to imaging parameters and aspects, so that, so that e.g.

$$Im = \begin{pmatrix} p \\ dq \\ ... \end{pmatrix},$$

where *p* represents parameters of the imaging sequence and *dq* parameters that describe data quality of the generated imaging data; *Sys* represents a sub-space vector relating to system aspects of the diagnostic imaging system, so that e.g.

$$Sys = \begin{pmatrix} RF \\ V \end{pmatrix},$$

where RF represents performance parameters of the radio frequency system and $\nabla$ represents performance parameters of the gradient system. The sub-space vector may further include aspects of the main magnetic field, such as actual field strength and a measure of the spatial field homogeneity, as well as other ambient circumstances such as temperature that may effect the quality of the operation of the diagnostic imaging system. The sub-space vector *Phys* represents physiology parameters of the subject to be imaged, so that for example

$$Phys = \begin{pmatrix} HR \\ Res \\ Haem \\ ... \end{pmatrix}$$

where HR represents heart rate, Res, respiratory patterns and *Haem* haematological details of the patient's blood. Also a measure of expected or actual body motion and body temperature of the patient to be examined may be included in the sub-space vector *Phys.*

From the measured physical and technical data, i.e. the measured state space vectors for the individual diagnostic imaging system, the status evaluation system returns a recognised evaluation of the operation of the diagnostic imaging system. The status evaluation system functions to return from the measured state space vector the location on or relative to a manifold in the multidimensional state space. In the multidimensional state space a plurality of manifolds are pre-defined. Each of the manifolds represents a level of acceptability of autonomous operation of the diagnostic imaging systems. These manifolds may in a simple example be defined by thresholding and windowing of the values of the components of the state space vector. For example, such thresholds may derive form human physiology, for example upper and lower limits of heart rate, blood pressure, oxygenation rate etc. In an other aspect the thresholds may relate to technical aspects of the performance limits of the magnetic resonance examination system, e.g. gradient coil temperature limits, power performance limits etc. The acceptability level of autonomous operations may vary between at one end full autonomous operation on the basis of an initiated imaging workflow to (possibly emergency) abortion of the current operation of the diagnostic imaging system. The location of the manifolds in state space may represent interactions of different patterns of values of multiple system or patient relates parameters. Normal acceptable patterns may be distinguished from abnormal uncomfortable of even hazardous patterns may be distinguished in alarming phase from sufficiently many measurements of these parameters. In a more advanced implementation, the acceptability level may take into account that acceptability ranges of one parameter (e.g. SAR, or gradient performance) may depend on other parameters, notably of imaging parameters such as diffusion weighting. Further, the allowable ranges for flip angles (i.e. RF transmit field amplitude) may be different for different types of image acquisitions, such as for multiple contrasts sequences such as MR-Stat or MR Fingerprinting or specific prepared contrasts like diffusion weighting or fMRI. The evaluation of the status may relate to the predicted impact on the current progress of an imaging workflow, such as a decision to abort the current image acquisition and initiate a re-acquisition. The evaluation may relate to evaluation of the data quality, such as the imaging data's signal-to-noise ratio or level of data corruptions or space recognise an associated acceptable level of autonomous operation of the individual diagnostic imaging systems. Such acceptable levels of autonomous operation may be defined by safety standards and by consensus in the diagnostic imaging communities and may vary among diagnostic imaging modalities. The evaluation may further relate to a predicted impact of the current operational state of the patient to be examined, such as acoustic noise level, SAR exposure or x-ray radiation dose. The status evaluation system in a more general sense determines predicted impact on the progress of the imaging procedure from the current operational state of the diagnostic imaging system. In a preferred implementation the status evaluation system is provided with a machine learning module, e.g. implemented by a trained deep learning network to return the predicted impact on the progress of the imaging procedure from the current operational state represented by the state space vector measured by the sensor system. Topological criteria in the state space may be employed to determine an appropriate trajectory in the state space to move the state space vector from a position having a low acceptability or even being hazardous to a topologically close position on a nearby manifold that has a good or at least better acceptability. The notion of topologically closeness may be defined in terms of similarity of diagnostic value in view of the clinical question at issue. For example, the closeness may represent similar image contrast, similar motion sensitivity, allowing for metal artefact correction to a similar extent. Also, system performance aspects that may be relevant for changing the state space vector from a current (undesired) position to a position on a manifold with higher acceptability may be taken into account. This may relate to hardware performance issues such as performance limits of the radio frequency system and the gradient system. Within this topological notion a steepest gradient descent strategy may be employed to time-efficiently change the state space vector's position and accordingly have the diagnostic system operate at a high(er) acceptability level while producing clinical relevant good quality image data.

With the predicted impact on the current imaging procedure there is associated an acceptable level of automation. The controller is configured to activate an action to control the individual diagnostic imaging systems to operate at the acceptable level of automation. In more detail, the controller functions on the basis of the determined impact on progress of the imaging procedure by the status evaluation system to continue the planned imaging workflow or to automatically revert to an alternative imaging procedure to avoid issues with predicted impact, prompt for assistance by a human operator, or in the event of a predicted imminent emergency, to abort the imaging procedure and initiate to remove the patient to be examined from the examination zone of the diagnostic imaging system. The controller further incorporates functions to provide a user interface representation of the evaluated status so that a human operator may overview the operational status over several diagnostic imaging systems, in fact of the entire group. Preferably a central user interface (UI) is provided to receive at its front-end the information representing the evaluated status of the diagnostic imaging systems and its back-end output provides a human perceptible information of these evaluated status. The central user interface and the automatic continuous evolution of the operational status of the diagnostic imaging systems achieves that the entire group of diagnostic imaging systems can be monitored by a small number, possibly only one, human operator. Moreover, there is no need for separate control consoles for each of the diagnostic imaging systems. In an advantageous implementation the user interface may comprise one or several mobile hand-held devices on which the evaluated operational status of the diagnostic image systems is displayed. Accordingly, at most very few (far less than the number of diagnostic imaging systems in the group) control rooms, or even only one single control room, to accommodate a single control console is needed, instead of separate control rooms for each of the diagnostic imaging systems. Thus, the arrangement for monitoring a group of diagnostic imaging systems of the invention supports much easier siting of the diagnostic imaging systems.

The arrangement for monitoring a group of diagnostic imaging systems of the invention enables to extend autonomous operation of the diagnostic imaging systems. Also autonomous operation is enabled to a higher degree in that upon a predicted issue, the controller may enable to amend the planned workflow so as to avoid or circumvent the predicted issue. This may be implemented in that in the event of prediction of motion artefacts, the image acquisition sequence as planned is replaced by an alternative image acquisition sequence that is less susceptible to motion while being able to acquire imaging that have proper contrast and image quality in view of the clinical question at issue. In the event of prediction of a too high anxiety of the (individual) patient to be examined due to annoying acoustic noise to change to a more silent version of the planned image acquisition. Further, fewer highly educated staff is required to support the operation of the diagnostic imaging systems as there is less or no need at all for human interpretation of the measured data acquired by the sensor system. Notably, need for human interpretation of the technical and physiological measurements, i.e. of the operation state space vector is obviated or at least reduced as this function is implemented in the deep learning, i.e. the trained neural network, of the status evaluation system.

The international application WO2012/052880 concerns a patient monitoring system comprising a probe system to acquire data from a patient, an evaluation module to determine a patient's state of anxiety based on the acquired data and an indicator module to feedback the determined state of anxiety to attending staff. The international application WO2012/052880 discloses the determination of a patient anxiety *per se,* without any indication to make use of the determined anxiety level to automate the operation of a diagnostic imaging procedure.

The present invention to a high degree automates the clinical workflow as detailed in an example of comparison of conventional workflow and a novel streamlined workflow supported by the operation of the diagnostic imaging system in an autonomous manner to a high degree. This is set out in the following Table 1.

| Table 1: Comparison of current workflow and proposed new simplified workflow | | |
|---|---|---|
| **Task / Type of interaction** | **Conventional state of the art workflow** | **Streamlined workflow of the invention** |
| Entering patient information (weight, sex, ...) | Manually at PC in the control room | Semi-automatically transferred from patient schedule (e.g. at central command centre), can be done in advance of the scan |
| Planning of scan | Manually at PC in the control room, partially automatic (SmartExam) | Fully automatic supported by an additional 3D camera in scanner room (e.g. above patient table) |
| Start scan | Manually at PC in the control room, partially at touch screen at scanner bore | At touch screen at scanner bore to start the scanning process, afterwards fully automated |
| Review data quality | MTRA / radiologist at PC in the control room / PACS | Automatically through AI, or in rare cases by MTRA / radiologist at PC e.g. at the central control room / PACS |
| Decision to do a rescan | MTRA / radiologist at PC in the control room / via PACS | Automatically based on artifact and patient schedule or MTRA / radiologist at PC in the control room, PACS |

(continued)

| Table 1: Comparison of current workflow and proposed new simplified workflow | | |
|---|---|---|
| **Task / Type of interaction** | **Conventional state of the art workflow** | **Streamlined workflow of the invention** |
| Error handling | at PC in the control room | At control touchscreen at the door to the MR system / in central command centre / remotely at customer support |

These and other aspects of the invention will be further elaborated with reference to the embodiments defined in the dependent Claims.

These and other aspects of the invention will be elucidated with reference to the embodiments described hereinafter and with reference to the accompanying drawing wherein

BRIEF DESCRIPTION OF THE DRAWINGS

Fig. 1 is a diagrammatic representation of a group of diagnostic imaging systems with an arrangement for monitoring of the invention

Fig. 2 is a diagrammatic representation of details of the arrangement for monitoring one of the diagnostic imaging systems and

Fig. 3 is a diagrammatic graphical representation of an example of the state spate of one of the diagnostic imaging systems.

DETAILED DESCRIPTION OF THE EMBODIMENTS

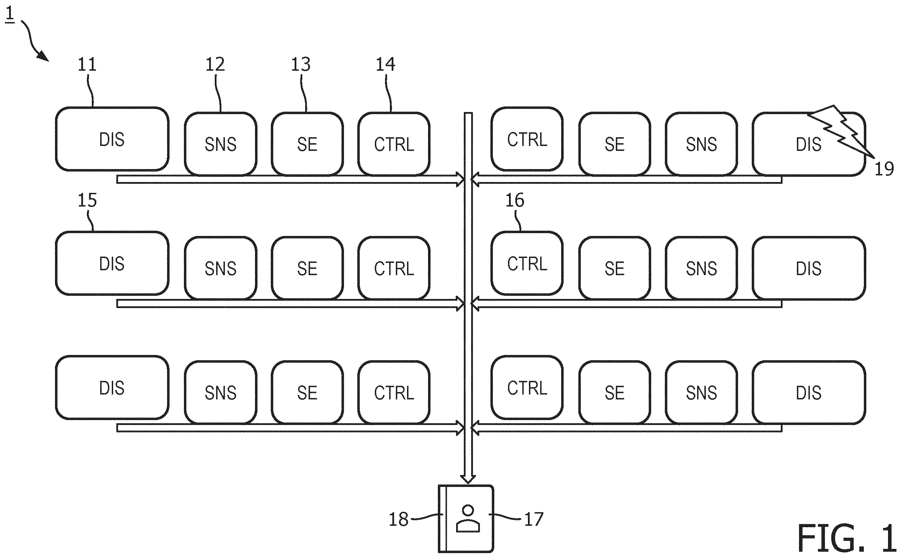

Fig. 1 is a diagrammatic representation of a group 1 of diagnostic imaging systems 11 with an arrangement (12,13,14,15,16,17) for monitoring of the invention. There are several diagnostic imaging systems (DIS) 11, for example magnetic resonance examination systems, computed tomography systems, x-ray imaging systems, nuclear imaging (PET or SPECT) systems. Each diagnostic imaging system 11 is associated with its sensor system (SNS) 12 coupled to it to measure the actual state space vector of the operational state of the diagnostic imaging system. The state space vector in the multi-dimensional state-space represents the operational status of the diagnostic imaging system. A state space evaluation system (SE) 13 is provided for the evaluation of the state space sensor of the diagnostic imaging system. The state space evaluation system 13 functions to recognise from the measured state-space vector's location in the multi-dimensional sate space an associated acceptable level of autonomous operation. In many situations, the measured state-space vector may be recognised to be on a manifold in state-space that is already associated with an acceptable level of automation. In such situations the diagnostic imaging system may continue to operate in its current operational status. In other situations, the state-space evaluation system 13 may recognise that the state-space vector does not lie on a manifold that is associated with an acceptable level of autonomous operation. In such a situation, the controller (CTRL) 14 is configured to activate an action to operate the diagnostic imaging system 11 in a way that has a state-space vector to a manifold close to the current state-space vector and adapt the state-space vector to be on that manifold of acceptable level of autonomous operations.

The sensor system 12 is coupled with the diagnostic imaging system over a local data bus 15 to receive status information of the diagnostic imaging system. The sensor system 12 outputs its sensed information over the local data bus 15 to the state evaluation system 13 that in turn outputs the measured state space vector the controller 14. The state space evaluation system 13 outputs instruction over a data bus 17 to its associated controller 14. Each individual diagnostic imaging system and the sensor system 12, the state space evaluation system 13 and the controller 14 associated with the diagnostic imaging system may be in communication over the local data bus 15. Furter each local data bus 15 for the respective diagnostic imaging systems is in communication with the location where there may be a human operator present over a central data exchange, that maybe a central data bus 17, or a wireless connection (Wi-Fi) with a mobile device held by the human operator, while moving among eh diagnostic imaging system of the group.

The controller may control the diagnostic imaging system over the data bus 17 to adapt its operational state to over a central data bus 18 inform the human operator on the operational state of the diagnostic imaging system 11 and/or prompt a warning signal over the communication channel 16 to a location 17 of an interface 18 for a human operator. The human operator may be mobile and provided with a communication device such as a tablet computer or a mobile telephone to receive the warning signal and communicate with the controller of the diagnostic system at issue to appropriately intervene in the diagnostic imaging system's operation. The communication from the controller to the interface for a human operator may be implemented at least in part as a wireless (e. g. Wi-Fi, Bluetooth,...) connection that allows the human operator to move about the multiple diagnostic imaging systems.

The concept of 'closeness' in the framework of the invention is associated with diagnostic similarity of the image content produced. For a magnetic resonance examination system closes state-space vectors may be associated with similar image contrasts. Closeness may also be associated with other image aspects or groups of image aspects, such as spatial resolution, contrast resolution, expected artefact levels, signal-to-noise ratio, contrast-to-noise ratio etc. The manifolds in state space are associated with various levels of acceptable levels of autonomous operation, while within a manifold state-space vectors may be associated with a variety of image aspects. The controller is configured to adapt a current state space vector that is found to lie outside a manifold to a state-space vector on a manifold and with image aspects that are near those of the current state space vector. Accordingly, the controller enables to have the diagnostic imaging system to continue autonomous operation, while maintaining the required image aspects. There may be technical situations that there is not an acceptable state space vector on manifold and having acceptable image aspects. The controller is configured to in such a situation to issue a warning over the common data exchange 16 to a human operator, and may also interrupt the imaging process to warrant safety of the patient to be examined. A control communication channel 16 is provided over which the controllers may communicate to a location 17 where there is a human operator to oversee the entire group of to a high degree of autonomously operating imaging systems.

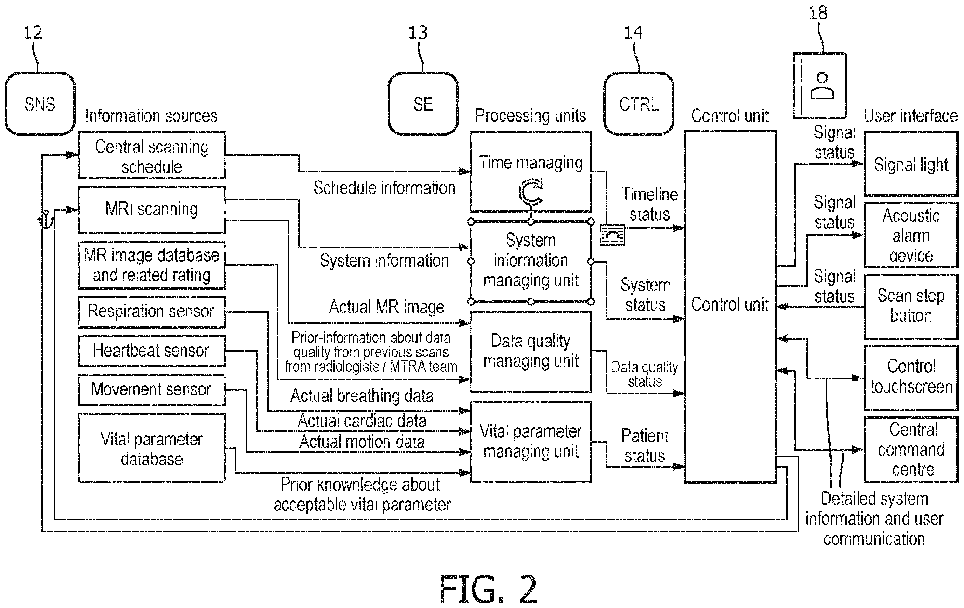

Fig. 2 is a diagrammatic representation of details of the arrangement as set forth in Fig. 1 for monitoring one of the diagnostic imaging systems 11. Fig. 2 shows more details of the functionalities of the sensor system 12, the state space evaluation system 13 and the controller 14. Further details are provided on the user interface for the human operator. The user interface may be implemented on the operator's mobile device. In particular, Fig. 2 shows several examples of sensors to measure the state space vector at which the diagnostic imaging system operates. Some of the state space vector elements may relate to the physiology of the patient to be examined while in the examination zone of the diagnostic imaging system. The state space evaluation systems 13 are formed as processing units for the evaluation of several parameters and assess if these correspond to a state space vector on a manifold that allows for a high level of autonomous operation of the diagnostic imaging system. The controller communicates status details as mentioned in the diagram of Fig. 2 to the user interfaces to inform the human operator. The controller may also adjust the operational state of the diagnostic imaging system. Alternatively, the controller may receive control commands from the user interface entered by the human operator to control the diagnostic imaging system to adjust is operational state or in the event of an emergency cease to acquire image data and even to allow to remove the patient to be examined immediately. For example, a warning light 19 or an acoustic alarm 19 may be provided at each of the diagnostic imaging systems. The controller 14 associated with the diagnostic imaging system may together with issuing a warning signal to the human operator also activate the local warning light 18 or acoustic signal to guide the human operator to the diagnostic imaging system that has an issue that needs human intervention.

In more detail:

The sensor systems 12 acquire data from several information sources:
The information input of the system is provided by sensors (respiration, heartbeat and movement), the central scanning schedule and by information stored in databases.

Central scanning schedule: Uses input from the hospital's scanning schedule and provides information about the daily scan plan to the time managing unit.

MRI system: Provides images and related data as well as system information (warnings/errors). Warnings and errors are already managed in current MRI systems; therefore, the information should be forwarded.

MR image database and related rating: During the normal image review process, radiologists could rate image quality. This information together with the image data could be used to train a neural network (locally) or a global MR data base can be used to train the network which is part of the data quality managing unit.

Respiration, heartbeat and movement sensor: Detect signals form patient and provide information for the vital parameter managing unit. These sensors already exist in MR systems, but the signals are currently not used with the focus to indicate the patient's condition rather they are used for triggering and gating of sequences.

Vital parameter database: based on prior knowledge and experiments a range of acceptable data values can be defined as input for the vital parameter managing unit.

The state space evaluation is carried out by processing units:
Time managing unit: To manage how much time the system is ahead or behind the schedule. This information will be transferred to the control unit to decide how much rescans can be done in case of bad data quality. In addition, it can be used to speed up the system (e. g. reducing resolution or omitting sequences) to bring it back to schedule automatically, or by passing the information through the control unit to the central command centre so that necessary interventions can be done by the personnel.

System information managing unit: Error and warnings should be managed to pass relevant information either to the central command centre or to the control touchscreen. In addition, the signal light should be used as an interface to communicate the system status to the MR users.

Data quality managing unit: Today an often-reported challenge in routing clinical scans is that issues with data quality or artifacts needed to be detected while the patient is on the patient table. This needs experienced staff or a radiologist. In addition, reviewing the images is difficult to organize since it requires time to carefully assess the image quality. Part of this proposal therefore is a trained deep learning convolutional neural network to identify insufficient image quality. The system could be trained on site with information which is presented by the radiologists by indicating the data quality on a e. g. 3-point Likert scale (3 = very good, 2 = acceptable, 1 = inacceptable). Depending on the rating of local radiologists separated for different body regions and sequence type the system should be able to identify bad or inacceptable data quality to prepare for a rescan. If a rescan is necessary, this should be indicated as an orange or red light supplemented by detailed information about the reason of the rescan. It can be advantageous to request the permission for an automatic rescan from the MR user via the control touchscreen or the central command centre, since the patient may have difficulties with an extended scan time.

On the basis of good data sets (i. e. without severe subject motion) and simulation of several images with subject movement by manipulating k-space data the deep learning convolutional neural network may be trained to identify insufficient image quality. With this approach thousands of images could be generated from very few real exams.

Vital parameter managing unit: Based on sensor data and the vital parameter database the vital parameter managing unit can decide if something is not going well with the patient. In addition, increased patient movement can be detected, and the information can be transferred so that the user can be informed.

The controllers 14 may have specific functions of:
Within the control unit the information comes together, and a dedicated algorithm is used to process information presented to the user via signal light, control touchscreen and central command centre. Furthermore, the unit should be able to communicate with the MRI scanner to change the scan protocol or to perform automatic rescans.

User interface (sub) systems may be provided at a location of the human operator and near the diagnostic imaging system:
Signal light: The signal light should be installed in a way that it can be easily seen from outside of the scanner room (see Figure 1). Table 2 provides an overview about possible intuitive light signals.

Acoustic alarm device: To indicate urgency, acoustic alarms could help gain attention.

Control Touchscreen: In a current MR system setting a computer monitor, a mouse and a keyboard is used to interact with the system. In the future, when the system become more autonomous, this set up may be reduced to use a simple control touchscreen close to the system, while the more complex interactions are performed from the central command centre.

Central command centre: In particular, at locations with more than one MRI system a central command centre is used, where conventionally MRI systems have their own control room. The command centre can be used e. g. to check image quality, to shorten the scan protocol in case the system is behind the scan schedule, or if additional sequences are needed. In such a setup well-trained stuff is only needed at the central command centre and not for every MRI system.

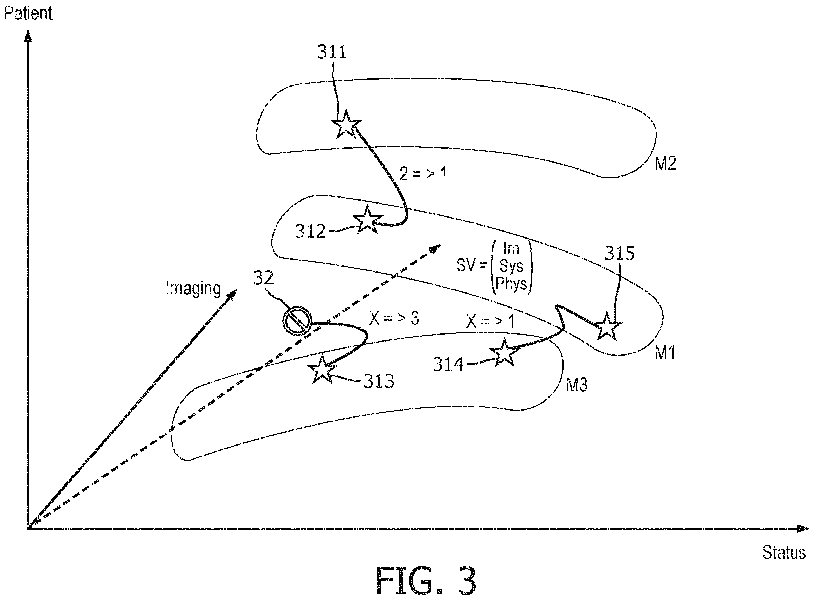

Fig. 3 is a diagrammatic graphical representation of an example of the state spate of one of the diagnostic imaging systems. In this example the operation state space of the diagnostic imaging system is spanned by imaging parameters and aspects, indicated as data quality, system aspects of the diagnostic imaging system and physiology parameters of the subject (patient to be examined) to be imaged. By way of example three manifolds of operational states are shown that allow to a high degree autonomous operation of the diagnostic imaging system. Also, efficient paths are shown in this multi-dimensional hyperspace of states, to move the state space vector between manifolds. Also shown are paths from a state space vector position outside a manifold, e. g. for which autonomous operation of the diagnostic imaging system is not recommended or even not allowed to a corresponding position on a manifold that produces similar image aspects now allowing to a high degree autonomous operation. Also shown is a state space vector having its position outside of the manifold. For such an unwanted state space vector a time-efficient path to a state space vector position that has similar imaging aspects but does lie on a manifold where autonomous operation is allowed to a high degree.

The example of the state space shown in Fig. 3 is spanned by system aspects, image aspects and patient aspects. Several manifolds (M1, M2, M3) are shown in which autonomous operation of the diagnostic imaging system is allowed to a high degree. That is for state space vectors on these manifolds, the diagnostic imaging system does not require to be monitored by a human operator and there is no need expected for human interventions. An example of a state space vector on the manifold M1 is shown as the bold dashed arrow *SV*. Further examples of state space locations on the manifolds are shown (311-315) as well as efficient paths between state space locations on respective manifolds labelled as (2=>1, 3=>1). These paths move the state space vector between manifold that allow autonomous operation of the diagnostic imaging system. These paths in state space may be determined by the responsiveness of the diagnostic imaging system's sub-systems to commands from the controller and on the performance limits of these sub-systems. On these manifolds the state space vector corresponds with different modes of operation and/or state of the patient to be examined. The different modes of operation may be associated with different imaging acquisition modes, e. g. imaging pulse sequences for a magnetic resonance examination system that may have different contrast encodings, different magnetisation preparations and various repetition times ($T_R$) and echo times ($T_E$) and associated with that different gradient waveforms and radio frequency transmit pulse shapes. The modes of operation may further be associated with system aspects, such as the main magnetic field homogeneity, non-linearities of the gradient system and operating temperatures of the radio frequency and gradient amplifiers and coils. For example, for a rampable magnet the system aspects may relate to the actual main magnetic field strength (Bo). The state space also represents aspects of the physiology of the patient to be examined, such as breathing rhythm, achievable breath-hold duration, heart rate , blood pressure and also body size and weight that may be relevant for determination of specific absorption rate (SAR) of transmitted radio frequency radiation for spin manipulation in the body of the patient to be examined. The paths between manifolds serve to move the state space vector of the diagnostic imaging system loaded with the patient to be examined between state spaces where the diagnostic imaging system may operate autonomously to a high degree, including the transfer from one state space position to another, that may be on a different manifold. An example of such an adaptation of the state space vector may be that the patient aspects include a high sensitivity for anxiety for loud noises (e. g. a caused by the gradient system of the magnetic resonance examination system) and the imaging aspects including a gradient waveform with steep trapezoid ramps. This may be sensed by the sensor system, e. g. from the detection of restless motion of the patient to be examined or from the questionnaire completed by the patient, this may evaluated by the state space evaluation system that then prompts the controller to shift the diagnostic imaging system to an acquisition sequence that process similar image contrast at less steep gradient ramps (i.e. reshape the trapezoids waveforms of the gradient pulses) which may be linked with a longer TR and longer scan time. In addition, the patient's condition can be influenced if a unpleasant patient situation is identified by targeted acoustic response from a person or a language model, different light settings, by music, or by changing the MR pulse sequence. Here a system could also learn users. However, sometimes real interaction with the patent may be necessary, e.g. if the patient is feeling pain.

An elaboration of the warning signals is presented in Table 2:

Table 2: *Signalling examples*

| Signal light colour | Acoustic alarm device | State | Comment |
|---|---|---|---|
| Red flashing | alarm | Critical situation in terms of patient safety (e.g. abnormal heart rate, breathing or motion) - immediate action required<br>e.g. irregular cardiac/respiratory signal, alarm button pressed by patient, critical system parameters (high quench risk) | Signal from vital parameters, acoustic alarm may be added |
| Red | silent | Critical situation but no safety issues - immediate action required:<br>e.g. insufficient image quality (e. g. due to subject motion), needed long rescan will cause a long delay, system error | |
| Orange | silent | Not ideal system performance - no immediate action required:<br>e. g. image data questionable, short delay because of an automatic rescan, slightly increased subject movement | Short time delay expected |
| Green blinking | silent | System scanning, everything runs as it is expected | Sufficient data quality, and no further delays |
| Green | silent | System ready for scan | Not technical problems, system on time |

Also shown in Fig. 3 is a not allowed state space position 32, i. e. outside any of the predefined manifolds. In such a not allowed state in state space the operational states of the diagnostic imaging system loaded with the patient to be examined is not allowed and my even form a hazardous situation, such as exceeding the SAR-limit on the patient to be examined or exceeding the performance specification of the radio frequency system or the gradient system of the magnetic resonance examination system. Also, simple safety issues, such as finger-pinching of the patient's finger between a patient carrier and the bore wall in the event of moving the table, the patient to be examined having lost the nurse-call button, or the patient's heart rate or blood pressure changing beyond a pre-set acceptable range. For such as state space vector position, the monitoring arrangement may search for a path to a state space position on a close-by manifold, where image data acquisition may be continued at a similar diagnostic value. In the event such a path is not found by the state space evaluation system 13, this is notified to the controller 14 that then issues a warning over the communication channel 16 to alert the human operator for a need for human intervention. Also, the controller 14 may activate the local warning light or acoustic alarm 19 to assist the human operator as to at which diagnostic imaging system of the group human intervention is called for.

**Claims**

1. Arrangement for monitoring a group of diagnostic imaging systems, the arrangement comprising

   - a sensor system for measurement of data representative of a multi-dimensional operational state space vectors for the individual diagnostic imaging systems;
   - a status evaluation system for evaluation from the measured state space vectors, the location of the actual state space status of the diagnostic imaging systems in the multi-dimensional state space representing the diagnostic imaging systems' operational status and from the location in the multi-dimensional state space recognise an associated acceptable level of autonomous operation of the individual diagnostic imaging systems and
   - a controller to activate an action to control the individual diagnostic imaging systems to operate at the acceptable level of automation.

2. An arrangement for monitoring a group of diagnostic imaging systems as claimed in Claim 1, in which the status evaluation system is configured to recognise the actual location of the state space vector on one or more manifolds in the operational state space, wherein the respective manifolds are associated each with a level of acceptable autonomous operation of the individual diagnostic imaging systems.

3. An arrangement for monitoring a group of diagnostic imaging systems as claimed in anyone of Claims 1 or 2, wherein the acceptable levels of automation include at least (i) full autonomous operation, (ii) requiring attention in view of an issue associated with the evaluated actual state space status or (iii) requiring immediate interference in view of an issue associated with the evaluated actual state space status.

4. An arrangement for monitoring a group of diagnostic imaging systems as claimed in anyone of Claims 1 to 3, wherein

   - the controller is configured to derive a human readable representation of the evaluated locations of the state space vectors for the individual diagnostic imaging systems and a central user interface is provided for the group of diagnostic imaging system and
   - the central user interface to receive the human readable representation and at its back-end output the human readable representation of the state space vectors.

5. An arrangement for monitoring a group of diagnostic imaging systems as claimed in Claim 2, wherein the respective manifolds are at least associated respectively with at least (i) full autonomous operation, (ii) requiring action in view of an issue associated with the evaluated actual state space status or (iii) requiring immediate interference in view of an issue associated with the evaluated actual state space status.

6. An arrangement for monitoring a group of diagnostic imaging systems as claimed in any one of the preceding Claims in which that state space is spanned by at least (i) status of an imaging process ongoing in the individual diagnostic imaging systems and (ii) status of object to be imaged in in the individual diagnostic imaging systems' examination zones.

7. An arrangement for monitoring a group of diagnostic imaging systems as claimed in any one of the preceding Claims in which the status evaluation concerns representing operational status of system functions and expected impact on continuation of imaging processes and/or of an object.

8. An arrangement for monitoring a group of diagnostic imaging systems as claimed in any one of the preceding Claims in which the state space has dimensions representing various technical functions of the individual diagnostic imaging systems and various physiology date of individual subjects in the respective diagnostic imaging systems' examination zones.

FIG. 1

EP 4 481 421 A1

FIG. 2

FIG. 3

Europäisches
Patentamt

European
Patent Office

Office européen
des brevets

# EUROPEAN SEARCH REPORT

**Application Number**

EP 23 18 0827

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/265219 A1 (FEIWEIER THORSTEN [DE] ET AL) 24 September 2015 (2015-09-24) | 1-7 | INV. G01R33/54 |
| Y | * paragraphs [0004] - [0069]; claims 1-15; figure 1 * | 1-7 | G01R33/28 |
| X | US 2023/154597 A1 (HELLE MICHAEL GÜNTER [DE] ET AL) 18 May 2023 (2023-05-18) | 1-7 | |
| Y | * paragraphs [0009] - [0017], [0048] - [0084]; claims 1-13; figures 1-3 * | 1-7 | |
| X | US 2020/008703 A1 (ZELLER MARIO [DE]) 9 January 2020 (2020-01-09) | 1-7 | |
| Y | * paragraphs [0023] - [0075]; claims 1-20; figures 1,2 * | 1-7 | |
| X | US 2022/183649 A1 (XU YIWEN [CN] ET AL) 16 June 2022 (2022-06-16) | 1-4 | |
| Y | * paragraphs [0004] - [0090]; claims 1, 5; figures 1-5, 8 * | 1-4 | |
| Y | US 2022/137172 A1 (SPECKNER THORSTEN [DE] ET AL) 5 May 2022 (2022-05-05) * paragraphs [0021] - [0031], [0068] - [0086]; figures 1,3 * | 1-8 | **TECHNICAL FIELDS SEARCHED (IPC)** G01R A61B G16H |
| Y | US 2016/091586 A1 (BENNER THOMAS [DE] ET AL) 31 March 2016 (2016-03-31) * paragraphs [0009], [0011], [0016] - [0026], [0043], [0044], [0057] - [0085]; claims 1-11; figures 1,2 * | 8 | |

The present search report has been drawn up for all claims

| Place of search | Date of completion of the search | Examiner |
|---|---|---|
| Munich | 6 December 2023 | Faber-Jurk, Sonja |

EPO FORM 1503 03.82 (P04C01)

## ANNEX TO THE EUROPEAN SEARCH REPORT
## ON EUROPEAN PATENT APPLICATION NO.

EP 23 18 0827

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2023

| Patent document cited in search report | | | Publication date | Patent family member(s) | | | Publication date |
|---|---|---|---|---|---|---|---|
| US 2015265219 | A1 | | 24-09-2015 | EP | 2921100 | A1 | 23-09-2015 |
| | | | | US | 2015265219 | A1 | 24-09-2015 |
| US 2023154597 | A1 | | 18-05-2023 | CN | 115349151 | A | 15-11-2022 |
| | | | | EP | 3889969 | A1 | 06-10-2021 |
| | | | | EP | 4128264 | A1 | 08-02-2023 |
| | | | | US | 2023154597 | A1 | 18-05-2023 |
| | | | | WO | 2021198044 | A1 | 07-10-2021 |
| US 2020008703 | A1 | | 09-01-2020 | DE | 102018210973 | A1 | 09-01-2020 |
| | | | | US | 2020008703 | A1 | 09-01-2020 |
| US 2022183649 | A1 | | 16-06-2022 | US | 2018344281 | A1 | 06-12-2018 |
| | | | | US | 2022183649 | A1 | 16-06-2022 |
| US 2022137172 | A1 | | 05-05-2022 | DE | 102020213920 | A1 | 05-05-2022 |
| | | | | US | 2022137172 | A1 | 05-05-2022 |
| US 2016091586 | A1 | | 31-03-2016 | CN | 105455812 | A | 06-04-2016 |
| | | | | DE | 102014219782 | A1 | 31-03-2016 |
| | | | | KR | 20160038796 | A | 07-04-2016 |
| | | | | US | 2016091586 | A1 | 31-03-2016 |

EPO FORM P0459

For more details about this annex : see Official Journal of the European Patent Office, No. 12/82

## REFERENCES CITED IN THE DESCRIPTION

**Patent documents cited in the description**

- EP 3564962 A
- WO 2012052880 A